# EUROPEAN PATENT APPLICATION

(11) **EP 1 479 782 A1**
(43) Date of publication of application: **24.11.2004**
(21) Application number: 04252904.0
(22) Date of filing: 19.05.2004
(51) Int. Cl.: C12Q 1/68

(54) **Method and system for analysis of a variable splicing of mRNAs by array hybridization**

(30) Priority: 19.05.2003 US 441782
(71) Applicant: Agilent Technologies, Inc., Palo Alto, CA 94306 (US)
(72) Inventor: Sampas, Nicholas M., San Jose, CA 95125 (US); Ach, Robert A., San Francisco, CA 94131 (US)
(74) Representative: Tollett, Ian

(57) **Abstract**

A method and system for determining the sequence of nucleic-acid polymers that is particularly useful for identifying various combinations of subsequences of a longer nucleic-acid sequence. Positive probes, including tiling probes (1102), jump probes (1308), and exonic tiling probes (1202), are employed within a microarray, along with a number of different types of negative control probes, including deletion-negative-control probes, reverse-jump-negative-control probes (1708), exon-linker-negative-control probes (1804), and intron/exon-negative-control probes (1904). The different types of positive probes combined with the different types of negative control probes provide a more precise and less ambiguous determination of various subsequent combinations that, for example, result from post-transcriptional splicing of mRNA transcripts..

## Description

The present invention relates to a method for determining the sequence of a variant splicing product of an initial MRNA transcript and, in particular, to a method and system for analyzing the sequences of various mRNA post-transcriptional-splicing products produced by protein-and-RNA-mediated cleavage and splicing of initial mRNA transcription products.

The present invention is related to the analysis of post-transcriptional splicing products of mRNA. In a described embodiment, post-translational splicing products of mRNAs are analyzed using microarray technology. For this reason, background information related to nucleic acids and to microarray technologies is provided, immediately below.

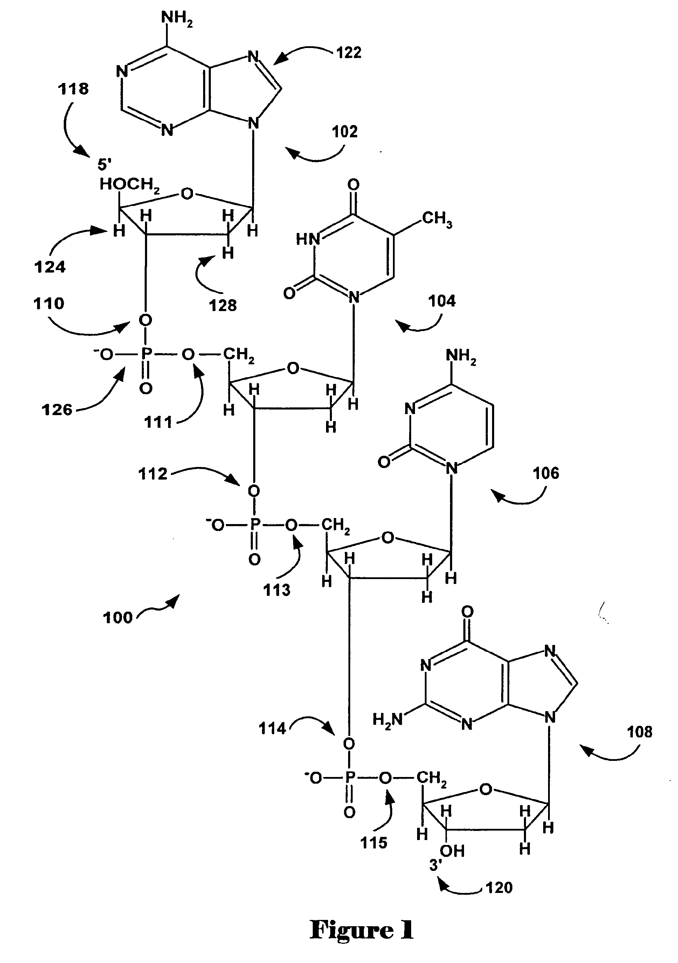

Deoxyribonucleic acid ("DNA") and ribonucleic acid ("RNA") are linear polymers, each synthesized from four different types of subunit molecules. The subunit molecules for DNA include: (1) deoxy-adenosine, abbreviated "A," a purine nucleoside; (2) deoxy-thymidine, abbreviated "T," a pyrimidine nucleoside; (3) deoxy-cytosine, abbreviated "C," a pyrimidine nucleoside; and (4) deoxy-guanosine, abbreviated "G," a purine nucleoside. The subunit molecules for RNA include: (1) adenosine, abbreviated "A," a purine nucleoside; (2) uracil, abbreviated "U," a pyrimidine nucleoside; (3) cytosine, abbreviated "C," a pyrimidine nucleoside; and (4) guanosine, abbreviated "G," a purine nucleoside. Figure 1 illustrates a short DNA polymer 100, called an oligomer, composed of the following subunits: (1) deoxy-adenosine 102; (2) deoxy-thymidine 104; (3) deoxy-cytosine 106; and (4) deoxy-guanosine 108. When phosphorylated, subunits of DNA and RNA molecules are called "nucleotides" and are linked together through phosphodiester bonds 110-115 to form DNA and RNA polymers. A linear DNA molecule, such as the oligomer shown in Figure 1, has a 5' end 118 and a 3' end 120. A DNA polymer can be chemically characterized by writing, in sequence from the 5' end to the 3' end, the single letter abbreviations for the nucleotide subunits that together compose the DNA polymer. For example, the oligomer 100 shown in Figure 1 can be chemically represented as "ATCG." A DNA nucleotide comprises a purine or pyrimidine base (e.g. adenine 122 of the deoxy-adenylate nucleotide 102), a deoxy-ribose sugar (e.g. deoxy-ribose 124 of the deoxy-adenylate nucleotide 102), and a phosphate group (e.g. phosphate 126) that links one nucleotide to another nucleotide in the DNA polymer. In RNA polymers, the nucleotides contain ribose sugars rather than deoxy-ribose sugars. In ribose, a hydroxyl group takes the place of the 2' hydrogen 128 in a DNA nucleotide. RNA polymers contain uridine nucleosides rather than the deoxy-thymidine nucleosides contained in DNA. The pyrimidine base uracil lacks a methyl group (130 in Figure 1) contained in the pyrimidine base thymine of deoxy-thymidine.

The DNA polymers that contain the organization information for living organisms occur in the nuclei of cells in pairs, forming double-stranded DNA helixes. One polymer of the pair is laid out in a 5' to 3' direction, and the other polymer of the pair is laid out in a 3' to 5' direction. The two DNA polymers in a double-stranded DNA helix are therefore described as being anti-parallel. The two DNA polymers, or strands, within a double-stranded DNA helix are bound to each other through attractive forces including hydrophobic interactions between stacked purine and pyrimidine bases and hydrogen bonding between purine and pyrimidine bases, the attractive forces emphasized by conformational constraints of DNA polymers. Because of a number of chemical and topographic constraints, double-stranded DNA helices are most stable when deoxy-adenylate subunits of one strand hydrogen bond to deoxy-thymidylate subunits of the other strand, and deoxy-guanylate subunits of one strand hydrogen bond to corresponding deoxy-cytidilate subunits of the other strand.

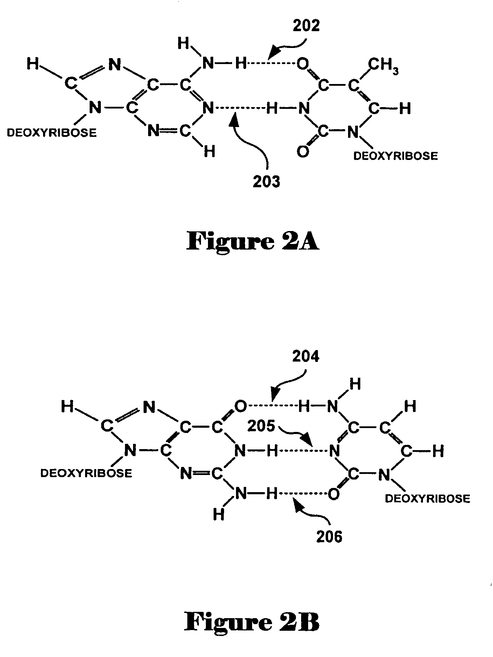

Figures 2A-B illustrate hydrogen bonding between the purine and pyrimidine bases of two anti-parallel DNA strands. Figure 2A shows hydrogen bonding between adenine and thymine bases of corresponding adenosine and thymidine subunits, and Figure 2B shows hydrogen bonding between guanine and cytosine bases of corresponding guanosine and cytosine subunits. Note that there are two hydrogen bonds 202 and 203 in the adenine/thymine base pair, and three hydrogen bonds 204-206 in the guanosine/cytosine base pair, as a result of which GC base pairs contribute greater thermodynamic stability to DNA duplexes than AT base pairs. AT and GC base pairs, illustrated in Figures 2A-B, are known as Watson-Crick ("WC") base pairs.

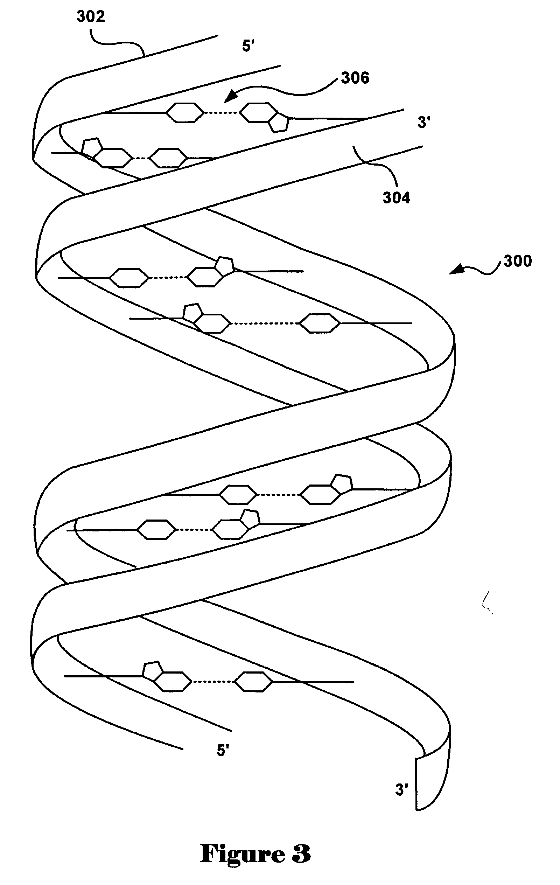

Two DNA strands linked together by hydrogen bonds form the familiar helix structure of a double-stranded DNA helix. Figure 3 illustrates a short section of a DNA double helix 300 comprising a first strand 302 and a second, anti-parallel strand 304. The ribbon-like strands in Figure 3 represent the deoxyribose and phosphate backbones of the two anti-parallel strands, with hydrogen-bonding purine and pyrimidine base pairs, such as base pair 306, interconnecting the two strands. Deoxy-guanylate subunits of one strand are generally paired with deoxy-cytidilate subunits from the other strand, and deoxy-thymidilate subunits in one strand are generally paired with deoxy-adenylate subunits from the other strand. However, non-WC base pairings may occur within double-stranded DNA.

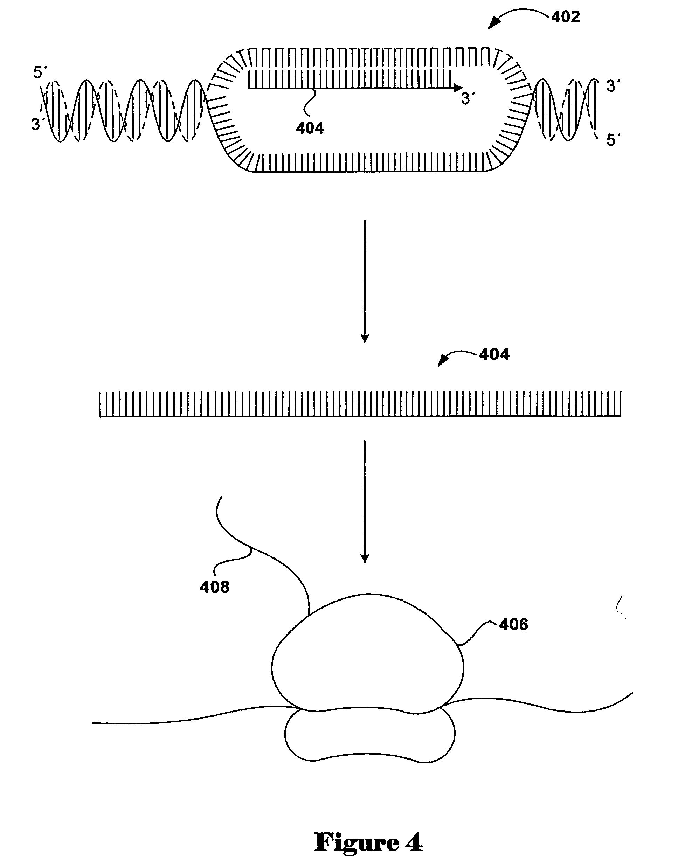

In order for the information encoded within DNA molecules to be used by cells within organisms, the DNA is first transcribed into mRNA. Figure 4 illustrates the transcription process. In Figure 4, a portion of a double-stranded DNA duplex 402 is shown to be locally unwound, and a single-stranded mRNA molecule 404 has been synthesized using one strand of the double-stranded DNA duplex as a template. Synthesis of mRNA transcripts complementary to a strand of a double-stranded DNA duplex is carried out in the cell by a large number of proteins that assist in unwinding the double-stranded DNA duplex, that transport nucleotide-triphosphate intermediates to the synthetic site, and by a DNA polymerase that condenses nucleotide-triphosphate intermediates to extend the growing mRNA polymer, the DNA polymerase selecting and condensing, at each nucleotide position, a nucleotide complementary to the nucleotide within the template strand of the double-stranded DNA duplex. The mRNA transcription product 404 then disassociates from the double-stranded DNA duplex 402 and is employed, depending on the DNA subsequence from which the mRNA transcript was transcribed, in different roles within a cell. The primary and first identified role for mRNA transcripts is for translation by a ribosomal complex, resulting in synthesis of a protein molecule. As shown in Figure 4, the ribosomal complex 406, comprising many tens of different protein and ribosomal RNA molecules, reads the information encoded within the mRNA transcript to produce the protein molecule 408 specified by the mRNA transcript. The each amino acid of the protein is which is encoded by nucleotide triplet within the mRNA.

Double-stranded DNA may be denatured, or converted into single stranded DNA, by changing the ionic strength of the solution containing the double-stranded DNA or by raising the temperature of the solution. Single-stranded DNA polymers may be renatured, or converted back into DNA duplexes, by reversing the denaturing conditions, for example by lowering the temperature of the solution containing complementary single-stranded DNA polymers. During renaturing or hybridization, complementary bases of anti-parallel DNA strands form WC base pairs in a cooperative fashion, leading to reannealing of the DNA duplex. Strictly A-T and G-C complementarity between anti-parallel polymers leads to the greatest thermodynamic stability, but partial complementarity including non-WC base pairing may also occur to produce relatively stable associations between partially-complementary polymers. In general, the longer the regions of consecutive WC base pairing between two nucleic acid polymers, the greater the stability of hybridization between the two polymers under renaturing conditions.

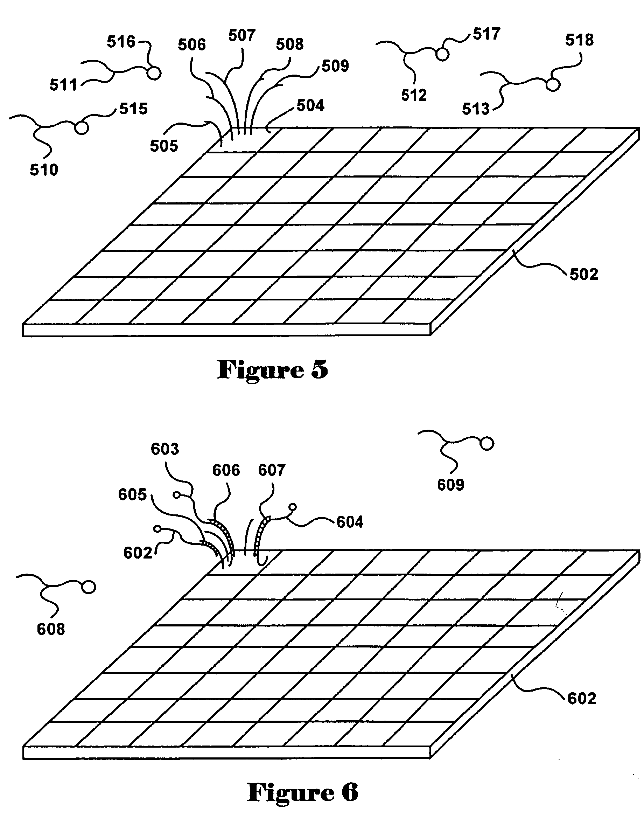

The ability to denature and renature double-stranded DNA has led to the development of many extremely powerful and discriminating assay technologies for identifying the presence of DNA and RNA polymers having particular base sequences or containing particular base subsequences within complex mixtures of different nucleic acid polymers, other biopolymers, and inorganic and organic chemical compounds. One such methodology is the array-based hybridization assay. Figures 5-8 illustrate the principle of the array-based hybridization assay. An array (502 in Figure 5) comprises a substrate upon which a regular pattern of features is prepared by various manufacturing processes. The array 502 in Figure 5, and in subsequent Figures 5-8, has a grid-like 2-dimensional pattern of square features, such as feature 504 shown in the upper left-hand comer of the array. Each feature of the array contains a large number of identical oligonucleotides covalently bound to the surface of the feature. These bound oligonucleotides are known as probes. In general, chemically distinct probes are bound to the different features of an array, so that each feature corresponds to a particular nucleotide sequence. In Figures 5-7, the principle of array-based hybridization assays is illustrated with respect to the single feature 504 to which a number of identical probes 505-509 are bound. In practice, each feature of the array contains a high density of such probes but, for the sake of clarity, only a subset of these are shown in Figures 5-7.

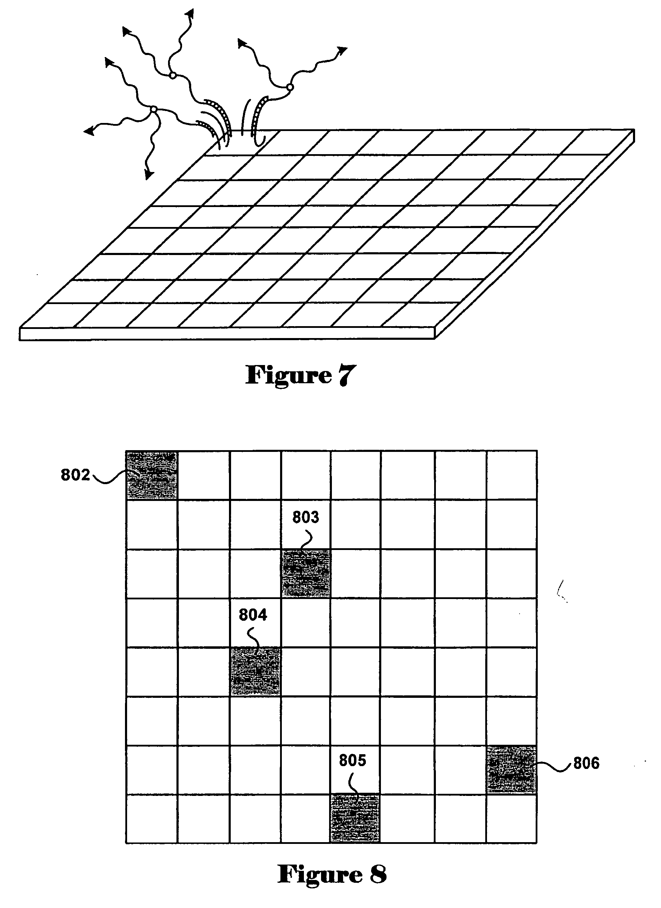

Once an array has been prepared, the array may be exposed to a sample solution of target DNA or RNA molecules (510-513 in Figure 5) labeled with fluorophores, chemoluminescent compounds, or radioactive atoms 515-518. Labeled target DNA or RNA hybridizes through base pairing interactions to the complementary probe DNA, synthesized on the surface of the array. Figure 6 shows a number of such target molecules 602-604 hybridized to complementary probes 605-607, which are in turn bound to the surface of the array 602. Targets, such as labeled DNA molecules 608 and 609, that do not contains nucleotide sequences complementary to any of the probes bound to array surface do not hybridize to generate stable duplexes and, as a result, tend to remain in solution. The sample solution is then rinsed from the surface of the array, washing away any unbound labeled DNA molecules. Finally, as shown in Figure 7, the bound labeled DNA molecules are detected via optical or radiometric scanning. Optical scanning involves exciting labels of bound labeled DNA molecules with electromagnetic radiation of appropriate frequency and detecting fluorescent emissions from the labels, or detecting light emitted from chemoluminescent labels. When radioisotope labels are employed, radiometric scanning can be used to detect the signal emitted from the hybridized features. Additional types of signals are also possible, including electrical signals generated by electrical properties of bound target molecules, magnetic properties of bound target molecules, and other such physical properties of bound target molecules that can produce a detectable signal. Optical, radiometric, or other types of scanning produce an analog or digital representation of the array as shown in Figure 8, with features to which labeled target molecules are hybridized similar to 806 optically or digitally differentiated from those features to which no labeled DNA molecules are bound. In other words, the analog or digital representation of a scanned array displays positive signals for features to which labeled DNA molecules are hybridized and displays negative features to which no, or an undetectably small number of, labeled DNA molecules are bound. Features displaying positive signals in the analog or digital representation indicate the presence of DNA molecules with complementary nucleotide sequences in the original sample solution. Moreover, the signal intensity produced by a feature is generally related to the amount of labeled DNA bound to the feature, in turn related to the concentration, in the sample to which the array was exposed, of labeled DNA complementary to the oligonucleotide within the feature.

Array-based hybridization techniques allow extremely complex solutions of DNA molecules to be analyzed in a single experiment. An array may contain from hundreds to tens of thousands of different oligonucleotide probes, allowing for the detection of a subset of complementary sequences from a complex pool of different target DNA or RNA polymers. In order to perform different sets of hybridization analyses, arrays containing different sets of bound oligonucleotides are manufactured by any of a number of complex manufacturing techniques. These techniques generally involve synthesizing the oligonucleotides within corresponding features of the array through a series of complex iterative synthetic steps.

An array may include any one-, two- or three-dimensional arrangement of addressable regions, called "features," bearing a particular chemical moiety or moieties, such as biopolymers, associated with that region. Array features are typically, but need not be, separated by intervening spaces. Array features contain probe molecules or other chemical entities bound to the array substrate. The probes are designed or selected to bind to target molecules or other chemical entities in sample solutions.

Any given array substrate may carry one, two, four or more arrays disposed on a front surface of the substrate. Depending upon the use, any or all of the arrays may be the same or different from one another and each may contain multiple spots or features. A typical array may contain more than ten, more than one hundred, more than one thousand, more ten thousand features, or even more than one hundred thousand features, in an area of less than 20 cm² or even less than 10 cm². For example, square features may have widths, or round feature may have diameters, in the range from 10 µm to 1.0 cm. In other embodiments each feature may have a width or diameter in the range of 1.0 µm to 1.0 mm, usually 5.0 µm to 500 µm, and more usually 10 µm to 200 µm. At least some, or all, of the features may be of different compositions (for example, when any repeats of each feature composition are excluded the remaining features may account for at least 5%, 10%, or 20% of the total number of features). Interfeature areas are typically, but not necessarily, present. Interfeature areas generally do not carry probe molecules. Such interfeature areas typically are present where the arrays are formed by processes involving drop deposition of reagents, but may not be present when, for example, photolithographic array fabrication processes are used. When present, interfeature areas can be of various sizes and configurations.

Each array may cover an area of less than 100 cm², or even less than 50 cm², 10 cm² or 1 cm². In many embodiments, the substrate carrying the one or more arrays will be shaped generally as a rectangular solid having a length of more than 4 mm and less than 1 m, usually more than 4 mm and less than 600 mm, more usually less than 400 mm; a width of more than 4 mm and less than 1 m, usually less than 500 mm and more usually less than 400 mm; and a thickness of more than 0.01 mm and less than 5.0 mm, usually more than 0.1 mm and less than 2 mm and more usually more than 0.2 and less than 1 mm. Other shapes are possible, as well. With arrays that are read by detecting fluorescence, the substrate may be of a material that emits low fluorescence upon illumination with the excitation light. Additionally in this situation, the substrate may be relatively transparent to reduce the absorption of the incident illuminating laser light and subsequent heating if the focused laser beam travels too slowly over a region. For example, substrate 10 may transmit at least 20%, or 50% (or even at least 70%, 90%, or 95%), of the illuminating light incident on the front as may be measured across the entire integrated spectrum of such illuminating light or alternatively at 532 nm or 633 nm.

Arrays can be fabricated using drop deposition from pulsejets of either polynucleotide precursor units (such as monomers) in the case of *in situ* fabrication, or the previously obtained polynucleotide. Such methods are described in detail in, for example, US 6,242,266, US 6,232,072, US 6,180,351, US 6,171,797, US 6,323,043, U.S. Patent Application Serial No. 09/302,898 filed April 30, 1999 by Caren et al., and the references cited therein. Other drop deposition methods can be used for fabrication, as previously described herein. Also, instead of drop deposition methods, photolithographic array fabrication methods may be used. Interfeature areas need not be present particularly when the arrays are made by photolithographic methods.

A molecular array is typically exposed to a sample including labeled target molecules, and the array is then read. Reading of the array may be accomplished by illuminating the array and reading the location and intensity of resulting fluorescence at multiple regions on each feature of the array. For example, a scanner may be used for this purpose, which is similar to the AGILENT MICROARRAY SCANNER manufactured by Agilent Technologies, Palo Alto, CA. Other suitable apparatus and methods are described in U.S. patent applications: Serial No. 10/087447 "Reading Dry Chemical Arrays Through The Substrate" by Corson et al., and Serial No. 09/846125 "Reading Multi-Featured Arrays" by Dorsel et al. However, arrays may be read by any other method or apparatus than the foregoing, with other reading methods including other optical techniques, such as detecting chemiluminescent or electroluminescent labels, or electrical techniques, for where each feature is provided with an electrode to detect hybridization at that feature in a manner disclosed in US 6,251,685, US 6,221,583 and elsewhere.

A result obtained from the reading followed by application of a method of the present invention, may be used in that form or may be further processed to generate a result such as that obtained by forming conclusions based on the pattern read from the array (such as whether or not a particular target sequence may have been present in the sample, or whether or not a pattern indicates a particular condition of an organism from which the sample came). A result of the reading (whether further processed or not) may be forwarded (such as by communication) to a remote location if desired, and received there for further use (such as further processing). When one item is indicated as being "remote" from another, this is referenced that the two items are at least in different buildings, and may be at least one mile, ten miles, or at least one hundred miles apart. "Communicating" information references transmitting the data representing that information as electrical signals over a suitable communication channel (for example, a private or public network). "Forwarding" an item refers to any means of getting that item from one location to the next, whether by physically transporting that item or otherwise (where that is possible) and includes, at least in the case of data, physically transporting a medium carrying the data or communicating the data.

As pointed out above, array-based assays can involve other types of biopolymers, synthetic polymers, and other types of chemical entities. A biopolymer is a polymer of one or more types of repeating units. Biopolymers are typically found in biological systems and particularly include polysaccharides, peptides, and polynucleotides, as well as their analogs such as those compounds composed of, or containing, amino acid analogs or non-amino-acid groups, or nucleotide analogs or non-nucleotide groups. This includes polynucleotides in which the conventional backbone has been replaced with a non-naturally occurring or synthetic backbone, and nucleic acids (or synthetic or naturally occurring analogs) in which one or more of the conventional bases has been replaced with a group (natural or synthetic) capable of participating in Watson-Crick type hydrogen bonding interactions. Polynucleotides include single or multiple stranded configurations, where one or more of the strands may or may not be completely aligned with another. For example, a "biopolymer" includes DNA (including cDNA), RNA, oligonucleotides, and PNA and other polynucleotides as described in US 5,948,902 and references cited therein, regardless of the source. An oligonucleotide is a nucleotide multimer of about 10 to 100 nucleotides in length, while a polynucleotide includes a nucleotide multimer having any number of nucleotides.

As an example of a non-nucleic-acid-based molecular array, one might attach protein antibodies to features of the array that would bind to soluble labeled antigens in a sample solution. Many other types of chemical assays may be facilitated by array technologies. For example, polysaccharides, glycoproteins, synthetic copolymers, including block coploymers, biopolymer-like polymers with synthetic or derivitized monomers or monomer linkages, and many other types of chemical or biochemical entities may serve as probe and target molecules for array-based analysis. A fundamental principle upon which arrays are based is that of specific recognition, by probe molecules affixed to the array, of target molecules, whether by sequence-mediated binding affinities, binding affinities based on conformational or topological properties of probe and target molecules, or binding affinities based on spatial distribution of electrical charge on the surfaces of target and probe molecules.

Scanning of a molecular array by an optical scanning device or radiometric scanning device generally produces a scanned image comprising a rectilinear grid of pixels, with each pixel having a corresponding signal intensity. These signal intensities are processed by an array-data-processing program that analyzes data scanned from an array to produce experimental or diagnostic results which are stored in a computer-readable medium, transferred to an intercommunicating entity via electronic signals, printed in a human-readable format, or otherwise made available for further use. Molecular array experiments can indicate precise gene-expression responses of organisms to drugs, other chemical and biological substances, environmental factors, and other effects. Molecular array experiments can also be used to diagnose disease, for gene sequencing, and for analytical chemistry. Processing of molecular array data can produce detailed chemical and biological analyses, disease diagnoses, and other information that can be stored in a computer-readable medium, transferred to an intercommunicating entity via electronic signals, printed in a human-readable format, or otherwise made available for further use.

Two or more data sets can be obtained from a single molecular array by scanning the molecular array for two or more signals. When optical scanning is used to detect fluorescent or chemiluminescent emission from chemophore labels, a first signal, or data set, may be generated by scanning the molecular at a first optical wavelength, and a second signal, or data set, may be generated by scanning the molecular at a second optical wavelength. Different signals may be obtained from a molecular array by radiometric scanning two detect radioactive emissions at two different energy levels. Target molecules may be labeled with either a first chromophore that emits light at a first wavelength, or a second chromophore that emits light at a second wavelength. Following hybridization, the molecular array can be scanned at the first wavelength to detect target molecules, labeled with the first chromophore, hybridized to features of the molecular array, and can then be scanned at the second wavelength to detect target molecules, labeled with the second chromophore, hybridized to the features of the molecular array. In one common molecular array system, the first chromophore emits light at a red visible-light wavelength, and the second chromophore emits light at a green, visible-light wavelength. The data set obtained from scanning the molecular array at the red wavelength is referred to as the "red signal," and the data set obtained from scanning the molecular array at the green wavelength is referred to as the "green signal." While it is common to use two different chromophores, it is possible to use three, four, or more different chromophores and to scan a molecular array at three, four, or more wavelengths to produce three, four, or more data sets.

Microarray-based techniques have been developed for sequencing nucleic acids or, in other words, determining the nucleotide sequences of nucleic acid polymers. These techniques generally employ probe nucleic-acid polymers complementary to small, overlapping subsequences within DNA molecules, allowing sequence information to be obtained from the pattern of microarray features to which fragments of a gene or other interesting DNA polymer hybridize. An interesting subproblem within the domain of nucleic-acid sequencing relates to determining the sequences of post-transcriptional splicing products of initial mRNA transcripts. While a number of microarray-based techniques have been proposed for addressing this problem domain, current microarray-based techniques do not provide sufficient discrimination and resolution for unambiguous identification of various splicing products. For this reason, researchers and microarray manufacturers have sought new, more discriminating methods and systems for rapid analysis of mRNA splicing products.

One embodiment of the present invention provides a method and system for determining the sequence of nucleic-acid polymers that is particularly useful for identifying various combinations of subsequences of a longer nucleic-acid sequence. The described embodiment employs microarrays that include positive probes, such as tiling probes, jump probes, and exonic tiling probes, along with a number of different types of negative control probes, including deletion-negative-control probes, reverse-jump-negative-control probes, exon-linker-negative-control probes, and intron/exon-negative-control probes. The different types of positive probes combined with the different types of negative control probes combine to provide a more precise and less ambiguous determination of the presence of various subsequence combinations within a post-transcriptional-splicing product of an initial mRNA transcript.

A number of preferred embodiments of the present invention will now be described with reference to the drawings, in which:-
Figure 1 illustrates a short DNA polymer.
Figure 2A shows hydrogen bonding between adenine and thymine bases of corresponding adenosine and thymidine subunits.
Figure 2B shows hydrogen bonding between guanine and cytosine bases of corresponding guanosine and cytosine subunits.
Figure 3 illustrates a short section of a DNA double helix.
Figure 4 illustrates the transcription process.
Figures 5-8 illustrate the principle of array-based hybridization assays.
Figure 9 illustrates post-transcriptional splicing of exons within a typical gene.
Figure 10 shows a simple hypothetical gene used in describing an embodiment of the present invention.
Figure 11 illustrates a first type of positive probe that may be included in a microarray used to analyze the variant splicing products of the hypothetical gene shown in Figure 10.
Figure 12 illustrates a second type of positive probe employed in microarrays designed for analyzing variant splicing products of the hypothetical gene shown in Figure 10.
Figure 13 illustrates a third type of positive probe employed in the analysis of variant splicing products of the hypothetical gene shown in Figure 10.
Figures 14A-D illustrate an example microarray containing the positive probes illustrated in Figures 11-13 and the results expected from scanning the example microarray when the target mRNA contains various combinations of exons 1-4 of the hypothetical gene shown in Figure 10.
Figure 15 illustrates a hypothetical feature-signal pattern produced by exposing the example microarray of Figure 14A to an unknown target splicing product of the exons with the hypothetical gene sequence shown in Figure 10.
Figures 16A-C illustrate a first type of negative control probe that can be used to resolve ambiguities in microarray data sets.
Figures 17A-C illustrate a second type of negative control probe that can be used to resolve ambiguities in a microarray data set.
Figures 18A-C illustrate a third type of negative control probe useful in resolving ambiguities in microarray data.
Figures 19A-D illustrate a final type of negative control probe.
Figure 20 illustrates inclusion of a number of negative control probes in the example array shown in Figure 15 in order to assist in determining whether or not the target molecule to which the example array is exposed contains exon 2.

One embodiment on the present invention is directed to a method and system for identifying subsequences of a nucleic-acid sequence that have been spliced together to form a splicing product of the nucleic-acid sequence. Particular advantage is gained from employing the present invention to analyze the post-transcriptional, variant splicing products of initial mRNA transcripts. The described embodiment, provided below, employs microarray-based techniques for analyzing these splicing products, although alternative method embodiments of the present invention may be employed in conjunction with a large number of different possible analytical techniques, including various solution-based techniques.

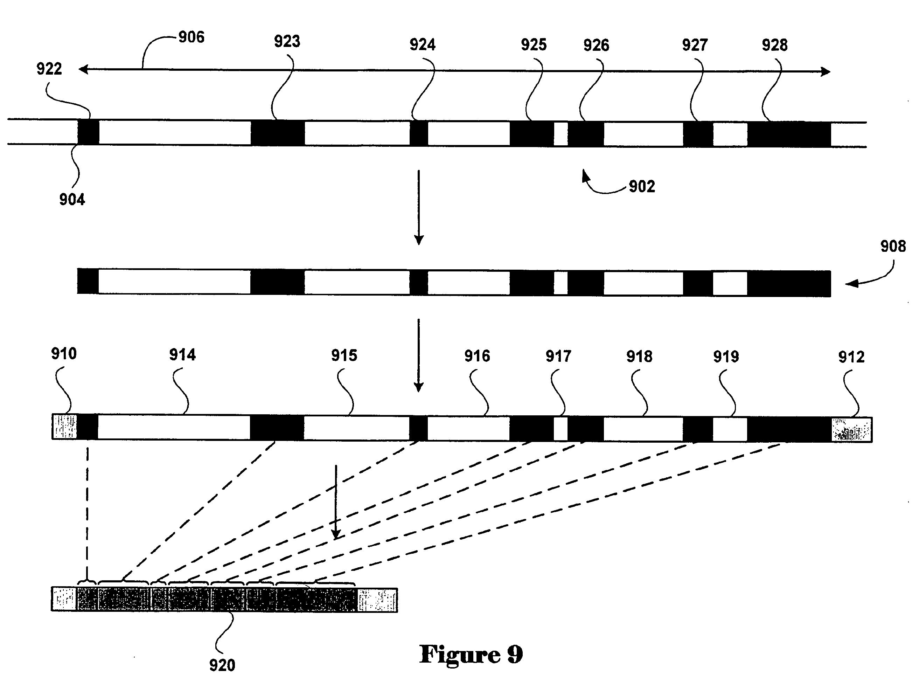

Figure 9 illustrates post-transcriptional splicing of exons within a typical gene. As shown in Figure 9, a gene 902 is a subsequence of a chromosomal DNA strand identified by a starting position 904 and a length 906 in nucleotide monomers, or bases. Transcription of a gene, as discussed above with reference to Figure 4, produces an mRNA transcript 908, a single-stranded RNA polymer that is complementary to a strand of the gene from which the mRNA transcript was transcribed. Following transcription, the mRNA transcript is chemically modified by various enzymes and ribozymes. Post-transcriptional processing of the initial mRNA transcript 908 includes the addition of a 7-methyl guanosine cap to the 5' end of the mRNA transcript 910, the addition of a poly-adenosine tail 912 to the 3' end of the initial mRNA transcript and, most significantly, excision of intron regions 914-919 from within the mRNA sequence to produce a final, capped, poly-adenylated, post-transcriptional-splicing product 920, which is the mature mRNA that is subsequently translated by ribosomal complexes to produce proteins. In the original gene 902, those subsequences of the gene 922-928 (shaded in Figure 9), transcription products of which are included in the final, mature mRNA 920, are referred to as "exons," while the subsequences that are excised from the initial mRNA transcription product 914-919 are referred to as "introns."

Although post-transcriptional processing of mRNA, and, in particular, post-transcriptional splicing of exons together to form a mature mRNA, was recognized only long after the fundamental gene-transcription and messenger-translation processes were elucidated, the post-transcriptional excision of introns and splicing together of exons to form mature mRNAs is now recognized as an extremely important component of the process by which genomic DNA directs the synthesis of proteins within cells. It has been learned that a single gene may produce a number of different gene products corresponding to cleavage and splicing together of various different combinations of exons within the original gene. Post-transcriptional processing thus may provide a many-fold increase in the genetic variability of an organism. Post-transcriptional processing may be, in part, controlled by elaborate feedback loops, so that the constellation of gene products produced within an organism at a particular point in time may be determined by the current needs of the organism for particular gene products. Thus, post-transcriptional processing of mRNAs provides a finely tunable and rapidly invoked mechanism for adaptability of an organism to internal and external conditions. For these reasons, recognizing the various post-transcriptional splicing variants of genes has become an important area of research.

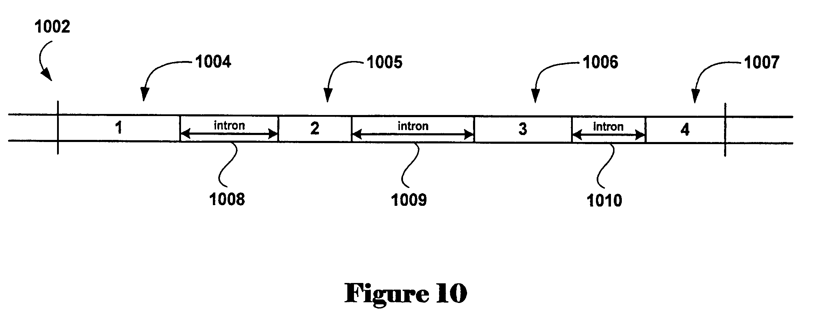

Figure 10 shows a simple hypothetical gene used in describing an embodiment of the present invention. The gene 1002 shown in Figure 10 comprises four exons 1004-1007 and three introns 1008-1010. In the following discussion of one embodiment of the present invention, it is assumed that two or more exons of the gene 1002 may be spliced together in their original sequence order to produce a final, mRNA transcript. Thus, it is assumed that a mature mRNA transcript of the hypothetical gene shown in Figure 10 may comprise one of the exon subsequence combinations {1,2}, {1,3}, {1,4}, {1,2,3}, {1,2,4}, {1,3,4}, {2,3}, {2,4}, {2,3,4}, and {3,4}. The methods of the present invention can be easily modified to analyze splicing products in which the exons are spliced together in orders other than their original sequence orders, by including additional positive probes and negative control probes.

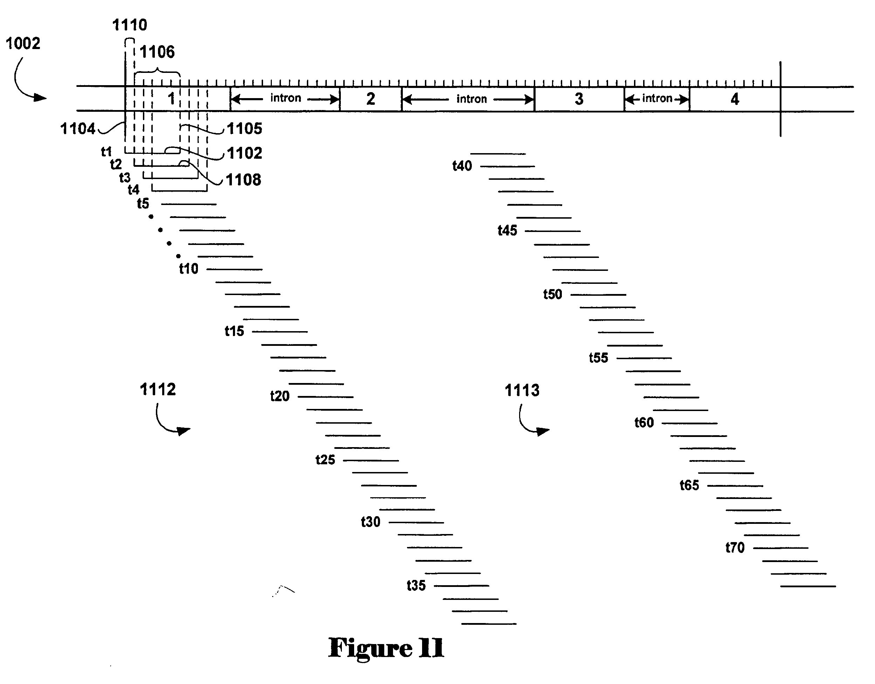

Figure 11 illustrates a first type of positive probe that may be included in a microarray used to analyze the splicing products of the hypothetical gene shown in Figure 10. The positive probes illustrated in Figure 11 are referred to as "tiling probes." The reference to tiling invokes the idea of overlaying the sequence of the gene 1002 with a series of overlapping subsequences, so that any particular nucleotide within the gene sequence is included within a number of shorter, tiling-probe subsequences. In Figure 11, the tiling probes are indicated by horizontal line segments, such as horizontal line segment 1102. As indicated in Figure 11 by dotted lines 1104-1105, the first tiling probe 1102, labeled *t1,* is a subsequence of nucleotides complementary to a first, equal-numbered subsequence 1106 within the gene sequence 1002. In Figure 11, each successive tiling probe is diagonally offset from a preceding tiling probe and from a succeeding tiling probe. For example, tiling probe t2 1108 is offset by a number of nucleotide subunits 1110, in the rightward direction from tiling probe *t1* 1102, and thus tiling probe *t2* 1108 is shown below, and offset to the right from, tiling probe *t1* 1102. The successive, overlapping tiling probes are thus shown as two diagonal columns 1112-1113 in Figure 11, with the tiling probes spanning the entire sequence of the hypothetical gene 1002. In one experiment, tiling probes of length 60 were used, with a 10-nucleotide-subunit offset between successive tiling probes. For tiling probes complementary to interior subsequences of the hypothetical gene 1002, each tiling probe is overlapped by five preceding tiling probes and five succeeding tiling probes. If a target mature mRNA is hybridized to a microarray containing the tiling probes illustrated in Figure 11, then the features containing tiling probes that significantly overlap the exon regions of the hypothetical gene sequence 1002 should produce signals when the microarray is scanned, while tiling probes that predominately overlap intron regions should exhibit low signal or no signal when scanned.

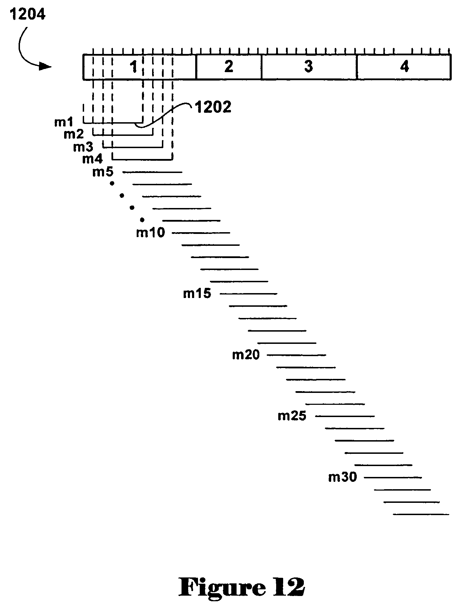

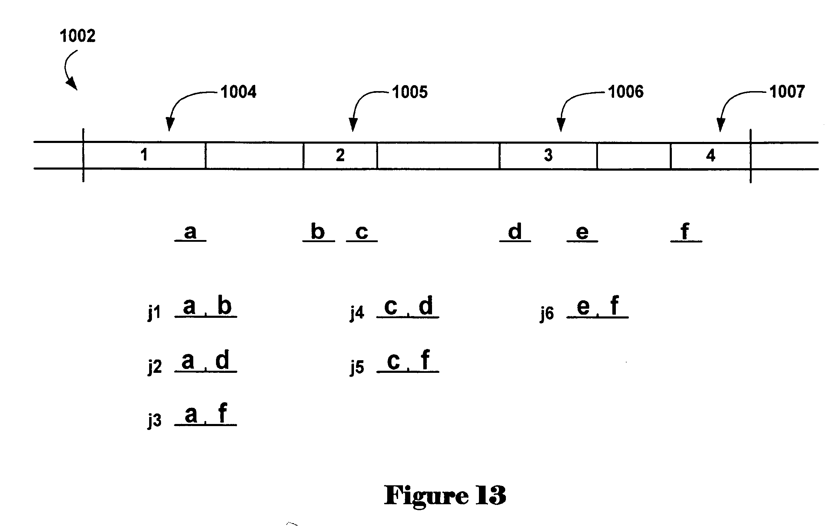

It should be noted that only the complementary subsequence portion of the tiling probes is shown in Figure 11 and in following Figures 12 and 13. In practice, additional nucleotides are included at one end of the complementary subsequence portion of probes in order to serve as linkers for linking the complementary subsequence portion of the probes to the substrate of a microarray. These linker stretches may be poly-A or poly-T subsequences, or may be constructed from synthetic, non-biologically occurring nucleotides or other polymeric materials and compounds.

Figure 12 illustrates a second type of positive probe employed in microarrays designed for analyzing variant splicing products of the hypothetical gene shown in Figure 10. The positive probes shown in Figure 12 are referred to as "exonic tiling probes." These tiling probes are constructed to overlap the in-order splicing product containing all of the exons within the hypothetical gene. Figure 12 uses the same illustration conventions as used in Figure 11, with the exonic tiling probes indicated by horizontal line segments, such as horizontal line segment 1202, below a representation of the hypothetical splicing product of exons 1-4 1204. Note that, in Figure 12, the exonic tiling probes are labeled starting with label *m1* for the first exonic tiling probe 1202. In alternate embodiments, exonic tiling probes complementary to reverse-order sequencing of the exons may be also used, in the case that reverse-order splicing of exons is anticipated in the post-transcriptional splicing products of the hypothetical gene.

Figure 13 illustrates a third type of positive probe employed in the analysis of variant splicing products of the hypothetical gene shown in Figure 10. The positive probes illustrated in Figure 13 are formed by combining initial and terminal subsequences of exons in sequence order. In the hypothetical gene of Figure 10 1002, there are four exons referred to as exons 1, 2, 3, and 4 1004-1007. Six exon subsequences 1302-1307 are used in pair-wise combinations to produce the two-exon-subsequence splice products 1308-1313 referred to as jump probes *j1* through *j6.* These jump probes thus are complementary to potential splice points at which two exons are joined together to form a final, mature, mRNA post-transcriptional-splicing product that serves as a target for the probes. Thus, for example, if the final mRNA post-transcriptional splicing product comprises exons 1, 3, and 4, then the jump probes *j2* and *j6* should hybridize to the target mRNA, while the remaining jump probes should hybridize only weakly, if at all.

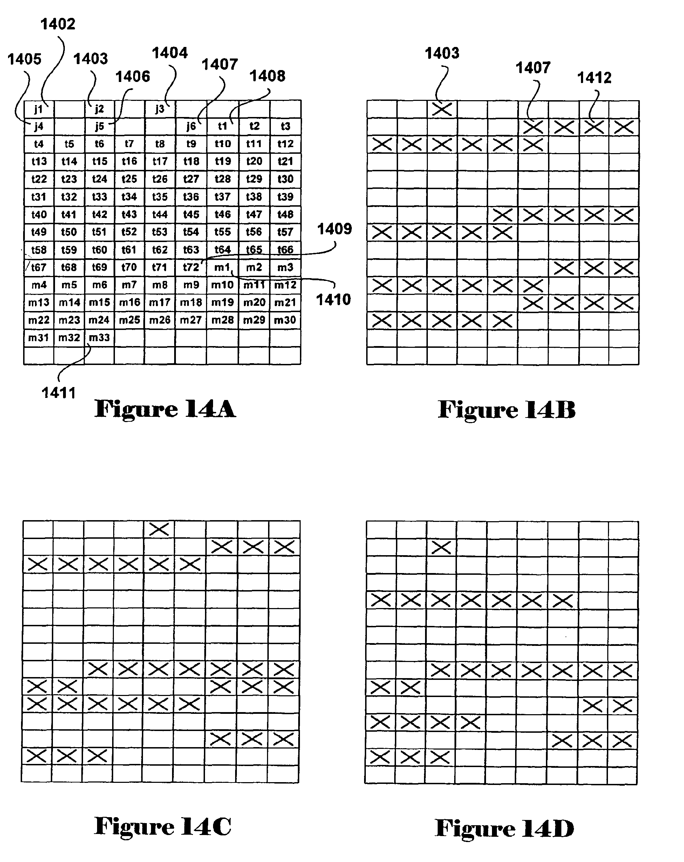

Figures 14A-D illustrate an example microarray containing the positive probes illustrated in Figures 11-13 and the results expected from scanning the example microarray when the target mRNA contains various combinations of exons 1-4 of the hypothetical gene shown in Figure 10. Figure 14A shows an example microarray with 15 rows and nine columns. Certain of the cells of the microarray are labeled with labels used to label the positive probes illustrated in Figures 11-13. For example, the jump probes *j1* through *j6* are contained in features (0,0) 1402, (0,2) 1403, (0,5) 1404, (1,0) 1405, (1,3) 1406, and (1,6) 1407, respectively, where the feature indices "(*x,y*)" refer to the feature in row *x* and column *y*. The tiling probes *t1* through *t72* illustrated in Figure 11 occur, in order, in the features starting with feature (1,7) 1408 and ending with feature (9,5) 1409. The exonic tiling probes *m1* through *m33*, illustrated in Figure 12, occur in successive features starting with feature (9,6) 1410 and ending with feature (13,2) 1411.

Figure 14B illustrates the signal pattern expected to be scanned from the example array shown in Figure 14A after the example array is exposed to a target molecule comprising a post-transcriptional splice product of the hypothetical gene shown in Figure 10 including exons 1, 3, and 4. Signals are expected from the features containing jump probes *j2* 1403, jump probe *j6* 1407, tiling probes that overlap exons 1, 3, and 4, and exonic tiling probes that overlap exons 1, 3, and 4. For example, tiling probe *t2* overlaps exon 1, and the feature corresponding to tiling probe *t*2 1412 therefore contains an "*x*" in Figure 14B indicating that a signal would be expected to be generated from this feature when the exposed microarray containing the feature is scanned. Figure 14C shows the pattern of signals expected to be scanned from example microarray following exposure of the example microarray to a target molecule comprising exons 1 and 4. Figure 14D shows the signal pattern expected to be generated by scanning the example microarray following exposure of example microarray to a target molecule comprising exons 2 and 4. Note that, in the idealized scans shown in Figures 14B-D, it is relatively straightforward to determine the presence and ordering of the exons within the target molecule to which the example microarray is exposed, in each case.

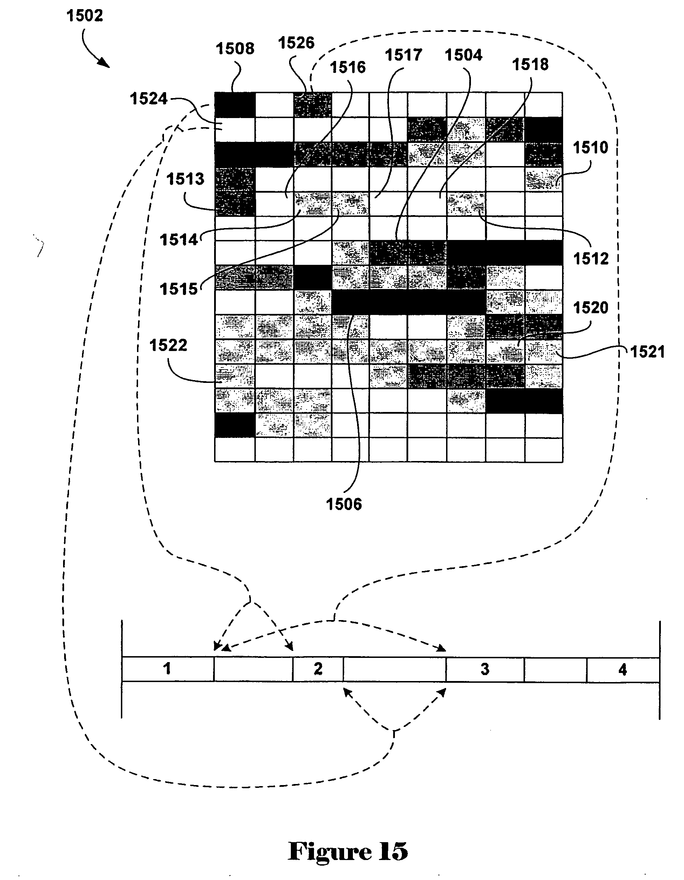

Unfortunately, the straightforward analysis illustrated in Figures 14B-D does not often occur in real experiments. Instead, various undesirable phenomena conspire to produce feature-signal patterns from scanned arrays that may offer alternative and ambiguous interpretation. Figure 15 illustrates a hypothetical feature-signal pattern produced by exposing the example microarray of Figure 14A to an unknown target splicing product of the exons of the hypothetical gene sequence shown in Figure 10. Examining the signal intensities scanned from the example array 1502 shown in Figure 15, where the darker shadings correspond to higher-intensity signals, it is readily apparent that the target splicing product of the hypothetical gene shown in Figure 10, to which the example array was exposed, includes exons 1, 3, and 4. Comparing the image of the scanned example array shown in Figure 15 with the ideal expected signal patterns of Figures 14B-C, it is readily observed that the block of tiling probes beginning with tiling probe *t1* (1407 in Figure 14B) exhibits relatively high-intensity signals in the scanned image shown in Figure 15, indicating the presence of exon 1. Similarly, the block of high-intensity signals starting with the cell corresponding to tiling probe *t44* 1504 and the block of high-intensity signals starting with the cell corresponding to tiling probe *t61* 1506 strongly indicate the presence of exons 3 and 4. However, it is unclear, upon examination of the scanned image of example array shown in Figure 15, whether or not exon 2 is present in the target splicing product. On one hand, the strong signal in the first cell of the example array 1508 corresponding to jump probe *j1* would seem to indicate that the subsequence symmetrically overlapping the splice junction between exons 1 and 2 was present in the target molecule. However, the cells starting with cell 1510 and ending with cell 1512, corresponding to the tiling probes *t21* through *t28* which overlap exon 2, show moderate-strength signals in cells 1513-1515, weak signals in cells 1510-1512, and no signal in cells 1516-1518. Similarly, the exonic tiling probes that overlap exon 2, including the probes *m11* through *m13,* corresponding to array cells 1520-1522, respectively, also show only weak signals. If exon 2 is in the target splicing product, then a strong signal should also be present for the jump probe *j4* in the array cell 1524. However, that cell shows no signal. Moreover, if exon 2 is in the target splicing product, then one would not expect a signal from the jump probe *j2,* corresponding to a splice junction between exons 1 and 3. However, in the scanned image shown in Figure 15, a fairly strong signal is found in the cell of the array 1526 corresponding to jump probe *j2.* Thus, examination of the scanned image of the example array provides ambiguous evidence as to the presence of exon 2 in the target splicing product to which the example array was exposed. The presence of strong signal in the cell 1508 corresponding to the jump probe *j1* indicates that exon 2 is present in the target splicing product, while the presence of a relatively strong signal in the cell 1526 corresponding to jump probe *j2* indicates that exon 2 may not be present. The weak-to-moderate strength signals in some of the tiling probes overlapping exon 2 also provides only an ambiguous and indeterminate indication of the presence of exon 2 in the target splicing product.

Various different types of unintended hybridization may contribute to ambiguous signals, such as those shown in the hypothetical example described with reference to Figure 15. It may be that, when an internal subsequence of an exon happens to have the sequence of a potential splice point, the jump probe constructed for that potential splice point give a false positive signal. Various types of non-specific binding may contribute to weak or even moderate-strength signals in the scanned image of an exposed array, even though the probes within the features producing the signal do not undergo sequence-specific hybridization with complementary subsequences of the target mRNA splicing product. Additional false positive signals may arise from contaminants, experimental errors, instrumental errors, and other such phenomena. False negative signals may also arise, although false negative signals are less likely to arise than false positive signals. The small example described above with reference to Figure 15 illustrates only a single ambiguity with respect to the presence of exon 2 in the target mRNA splicing product, but the arrays used for alternative splicing analysis may contain thousands to tens of thousands of features for testing many different potential combinations of a large number of exons in one or more mRNAs, and the resulting ambiguities may be complex and difficult to unravel.

For these reasons, a method has been sought for eliminating potential ambiguities from microarray data sets related to variant splicing analysis. It was determined that inclusion of different types of negative control probes can greatly facilitate precise determination of the presence of variant splicing products in sample solutions to which microarrays are exposed.

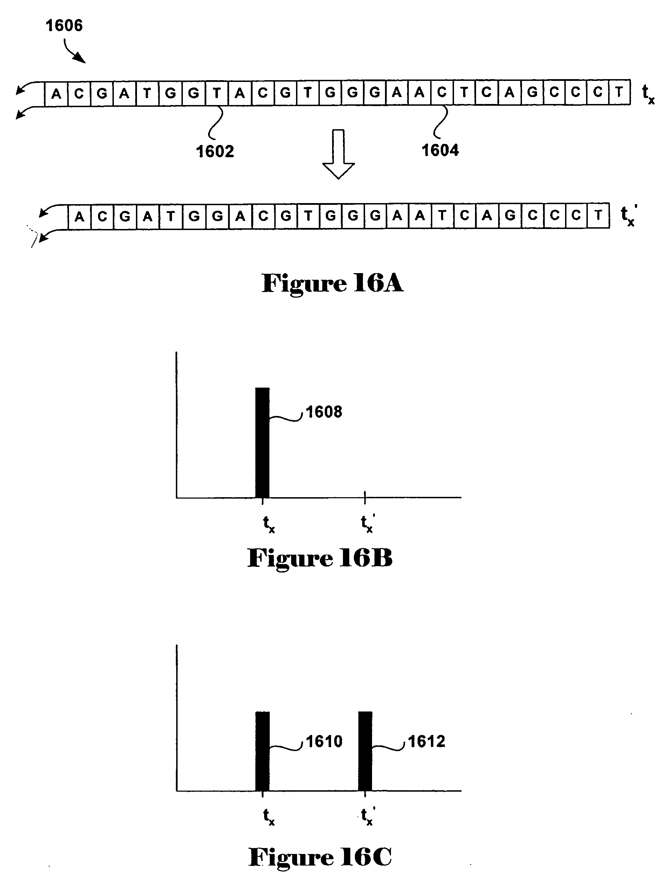

Figures 16A-C illustrate a first type of negative control probe that can be used to resolve ambiguities in microarray data sets. The first type of negative control probe is a deletion-negative-control probe. Figure 16A illustrates the creation of a deletion-negative-control probe *t*_{*x*}*'* from a tiling probe *t*_{*x*}*.* In the example shown in Figure 16A, two nucleotide subunits 1602 and 1604 are deleted from the 26-nucleotide-subunit tiling probe *t*_{*x*} 1606 to produce a 24-nucleotide-subunit tiling probe *t*_{*x*}*'.* In general, the deleted nucleotide subunits are distributed regularly throughout the tiling probe, so that the tiling probe sequence is transformed into a collection of contiguous subsequences of relatively uniform length demarcated by the deletion points. A target molecule that would have readily hybridized to the tiling probe via complementary based pairing would not be expected to so readily hybridize to the negative deletion-negative-control probe.

In general, hybridization of a target molecule to a probe is characterized by the melting point, or *t*_{*m*}*,* a temperature at which 50% of associated targets and probes disassociate. Raising the temperature above *t*_{*m*} drives the targets and probes to disassociate, and lowering the temperature below *t*_{*m*} facilitates a robust and long-term hybridization between the targets and complementary probes. Target and probes that have full complementarity, generally have higher *t*_{*m*} melting points than target and probe molecules having only partial complementarity, or that are non-specifically associated with one another. The longer the complementary target and probe molecules, the higher the *t*_{*m*}*.* Finally hybridized target and probes with higher proportions of G-C and C-G base pairs having higher *t*_{*m*} melting points. Tiling probes, jump probes, and exonic tiling probes may be constructed to exhibit a selected *t*_{*m*} for hybridization to complementary target subsequences. Such considerations dictate the nucleotide-subunit lengths of tiling probes, exonic tiling probes, and jump probes. Thus, a negative deletion-negative-control probe should exhibit a relatively low *t*_{*m*} and thus relatively low hybridization, to the target sequence of the corresponding positive probe from which the deletion-negative-control probe is created.

Figures 16B-C show plots of expected signal strength for the tiling probe *t*_{*x*} and the negative control probe *t*_{*x*}*'* when the sample solution contains target subsequences complementary to the tiling probe *t*_{*x*}*,* in the case of Figure 16B, and non-complementary but non-specifically hybridizing to the tiling probe in Figure 16C. Figures 16B-C, and similar figures to follow, plot the signal strength in a vertical direction for the positive probe and negative control probe situated along a horizontal axis. Thus, when the sample solution contains a target subsequence that specifically hybridizes to the tiling probe *t*_{*x*}*,* a relatively large signal 1608 should be scanned from the cell of an array containing the tiling probe. However, in general, little or no signal should be scanned from a negative control probe *t*_{*x*}*'* constructed from the tiling probe by removing nucleotide subunits at regular intervals, presuming that the temperature during exposure is maintained somewhat below the *t*_{*m*} for the expected tiling probe *t*_{*x*}/*target* hybridization, but above that for non-complementary association of the target subsequence and the negative deletion-negative-control probe *t*_{*x*}*'.* However, in the case that the tiling probe *t*_{*x*} binds through low-complementary or non-complementary association with target subsequences, then it may be expected that deletion of a few nucleotide subunits from the tiling probe *t*_{*x*} should not greatly affect the non-specific association, and therefore comparable signals should be obtained from the cells of an array containing both the tiling probe *t*_{*x*} and the negative deletion-negative-control probe *t*_{*x*}*'.* Figure 16C shows a situation in which the two signals 1610 and 1612 have comparable signal strengths. Thus, the signal obtained from a negative deletion-negative-control probe, such as the negative deletion-negative-control probe *t*_{*x*}*'* illustrated in Figure 16A, can be used in combination with the signal obtained from the corresponding positive probe, such as positive probe *t*_{*x*} in Figure 16A, to determine whether or not the signal produced by the positive control probe likely arises from non-specific hybridization or from specific hybridization based on complementary based pairings. Of course, it may also the case that a particular negative deletion-negative-control probe is fortuitously complementary to some other subsequence of a target molecule, but the likelihood of such fortuitous complementarities is relatively small, and can be managed by selecting positive probes of appropriate lengths. Note that negative deletion-negative-control probes can be constructed from any of the various types of positive probes.

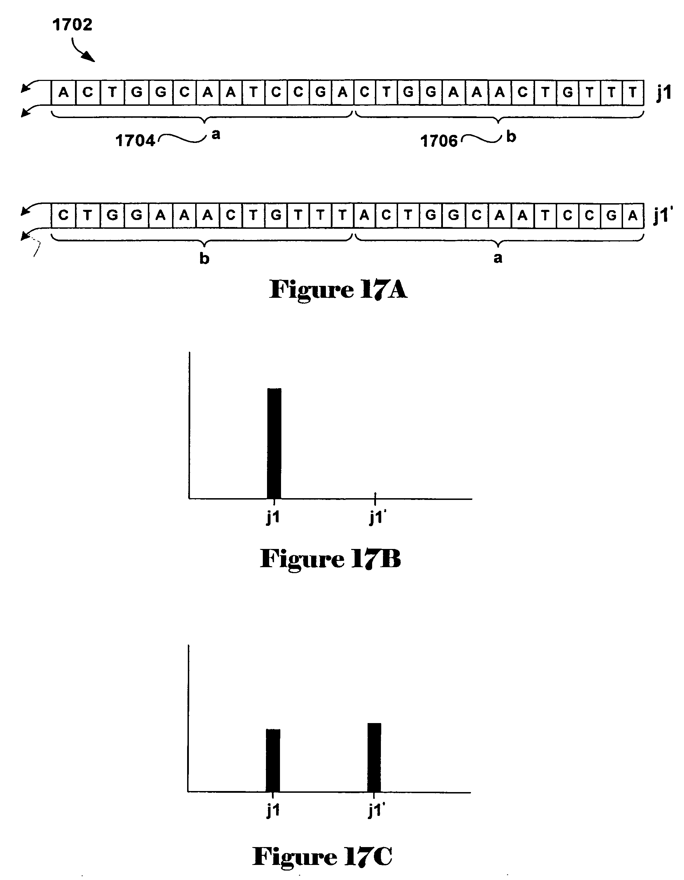

Figures 17A-C illustrate a second type of negative control probe that can be used to resolve ambiguities in a microarray data set. As shown in Figure 17A, a jump probe *j1* 1702 comprising a terminal subsequence "a" 1704 from a first exon and an initial subsequence "b" 1706 from a second exon may be converted into a reverse-jump-negative-control probe *j1'* 1708 by reversing the order of the terminal subsequence "a" and initial subsequence "b" within the negative reverse-jump-negative-control probe *j1'.* Figures 17B-C illustrate the expected signal strength from the positive jump probe *j1* and the reverse-jump-negative-control probe *j1'* when the positive jump probe *j1* hybridizes specifically to a target subsequence and when the positive jump probe *j1* hybridizes nonspecifically to a target subsequence, respectively. This can be seen in Figure 17B, when the positive jump probe *j1* hybridizes, through complementary base pair associations, to a target subsequence, indicating the presence of a splice point within the target subsequence. Reversing the terminal and initial subsequences within the positive jump probe *j1* should then produce a reverse-jump-negative-control probe *j1'* that hybridizes with a much lower *t*_{*m*} to only a portion of the target subsequence including the splice point. When hybridization is carried out at a temperature below the *t*_{*m*} for hybridization of the positive jump probe *j1* with the splice-point-containing subsequence, but significantly above the *t*_{*m*} for partial hybridization of a portion of the reverse-jump-negative-control probe *j1'* with a portion of the splice-point-containing target subsequence, then, as shown in Figure 17B, it would be expected that the signal produced by an array cell containing positive jump probe *j1* would be strong, while only a weak signal, or no signal, would be expected from the array cell containing the reverse-jump-negative-control probe *j1'.* By contrast, non-specific hybridization of various target subsequences to the positive jump probe *j1* should not be greatly affected by reversing the order of the initial and termination sequences of positive jump probe *j1* to produce the reverse-jump-negative-control probe *j1',* and thus, as shown in Figure 17C, cells containing both the positive jump probe and the reverse-jump-negative-control probe should produce comparable signals.

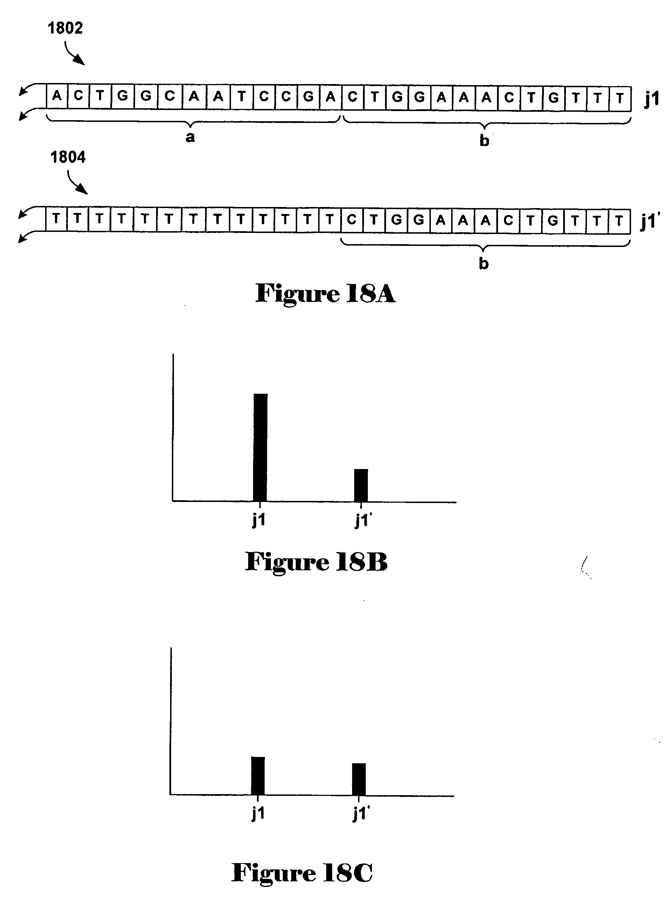

Figures 18A-C illustrate a third type of negative control probe useful in resolving ambiguities in microarray data. This third type of negative control probe also involves altering a positive jump probe. As shown in Figure 18A, a positive jump probe *j1* 1802 may be altered by substituting a poly-T sequence for one of the two subsequences "a" or "b" in the positive jump probe *j1* 1802 to produce a exon-linker-negative-control probe 1804. Although Figure 18A shows substitution of a poly-T sequence for the "a" subsequence, a different exon-linker-negative-control probe is obtained by substituting a poly-T sequence for the "b" subsequence. Note that, in general, the probe sequences illustrated in the figures, and described in the text, are linked to the microarray substrate through linker polymers, generally comprising poly-T or poly-A polymers. Thus, the exon-linker-negative-control probe 1804 essentially amounts to extending the length of the linker and decreasing the length of the target-specific probe sequence by a factor of two. As shown in Figures 18B-C, comparison of the signals produced by a positive jump probe *j1* and its corresponding exon-linker-negative-control probe *j1'* should show a much stronger signal strength from a feature containing the positive jump probe *j1* than from a feature containing the exon-linker-negative-control probe *j1',* since a longer specific hybridization through complementary based pairing produces a hybridized pair with a much higher *t*_{*m*}*.* Again, as shown in Figure 18C, non-specific associations within a positive probe and various target subsequences should be less affected by decreasing the length of the target-specific sequence portion of the probe.

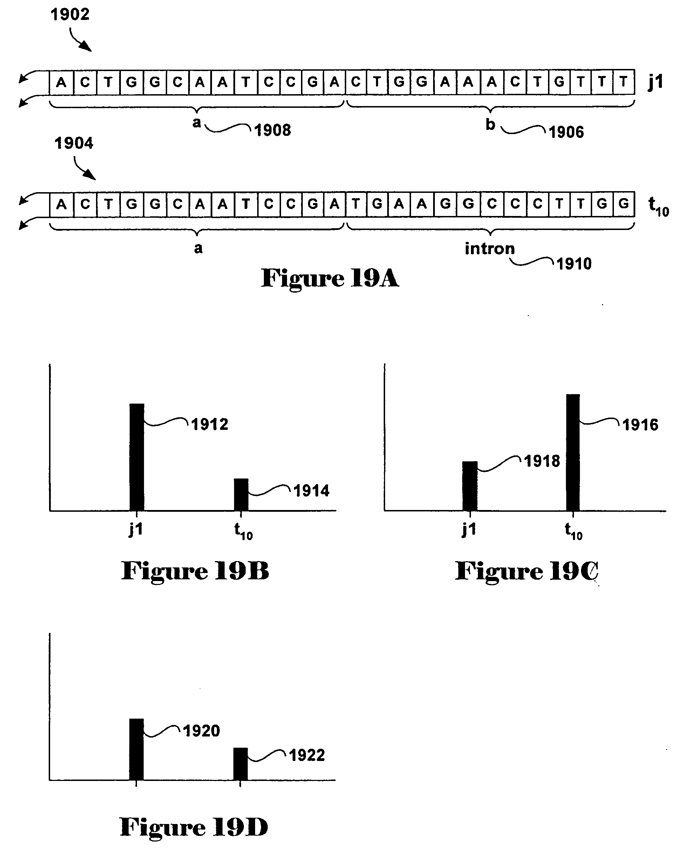

Figures 19A-D illustrate a final type of negative control probe. Again, as in the previous two described negative control probes, a positive jump probe *j1* 1902 is transformed into a negative control probe 1904. In this case, the initial subsequence "b" 1906 is replaced with the initial subsequence of the intron adjacent to the terminal subsequence "a" 1908 in the initial, unprocessed mRNA transcript, prior to intron excision and splicing. Note that, in the present example, this intron/exon-negative-control probe 1904 is identical to the tiling probe *"t10."* Thus, certain specific tiling probes can be alternatively considered to be intron/exon-negative-control probes for particular positive probes. Although not shown in Figure 19, a different intron/exon-negative-control probe can be obtained by substituting the terminal subsequence of the intron adjacent to initial subsequence "b" in the initial, unprocessed mRNA transcript for the terminal subsequence "a." As another example, the exonic tiling probes that span splice points may be similar or identical to corresponding jump probes, depending on the lengths of the terminal and initial subsequences from which the jump probe is constructed.

As shown in Figure 19B, when the positive jump probe *j1* is complementary to a subsequence of the final, splicing product containing the splice point contained in the positive jump probe, then the signal from the positive jump probe 1912 should be of significantly greater magnitude than any signal produced by the intron/exon-negative-control probe 1914. By contrast, the positive jump probe contains a potential splice point that is not found in the final, splicing product, then, as shown in Figure 19C, the signal produced by a microarray feature containing the intron/exon-negative-control probe 1916 should be greater than the signal, if any, produced by the positive jump probe 1918. As usual, should non-specific hybridization be responsible for the signal produced by the positive jump probe 1920, then the signal produced by the intron/exon-negative-control probe 1922 should be of comparable magnitude.

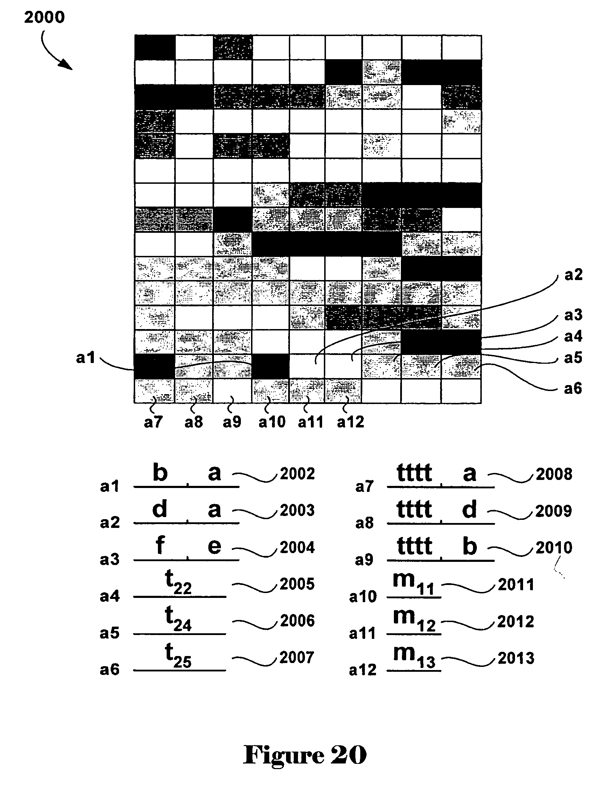

Figure 20 illustrates inclusion of a number of negative control probes in the example array shown in Figure 15 in order to assist in determining whether or not the target molecule to which the example array is exposed contains exon 2. The negative control probes added to the example array are shown below the scanned image of the array in Figure 20, and are labeled *a1* through *a12.* The negative control probes include three reverse-jump-negative-control probes 2002-2004, three deletion-negative-control probes based on tiling probes 2005-2007, three exon-linker-negative-control probes 2008-2010, and three negative deletion-negative-control probes 2011-2013 based on exonic tiling probes. The cells of the array 2000 containing the various added negative control probes *a1* through *a12* are indicated by labels "a1" through "a12" in Figure 20.

Consider the additional information provided by the negative control features. First, the relatively strong signal produced by the microarray cell containing negative control probe *a1* indicates that the strong signal produced by positive jump probe *j1* (1508 in Figure 15) is probably due to non-specific or unintended complementary hybridization of the positive jump probe to some subsequence within the target molecule. In other words, the relatively strong signals produced by positive jump probe *j1* and reverse-jump-negative-control probe *a1* corresponds to the signal-strength case shown in Figure 17C. Thus, negative control probe *a1* reveals that the strong signal from positive jump probe *j1* may be spurious, and not indicate the presence of an exon 1/exon 2 splice point in the post-transcriptional splicing product of the hypothetical gene. Furthermore, the absence of signal from the array cell containing the reverse-jump-negative-control probe *a2* reveals that the strong signal produced by the positive jump probe *j2 (*1526 in Figure 15) arises from the presence of the exon 1/exon 3 splice point within the target splicing product. The weak-to-moderate signals produced by cells corresponding to negative deletion-negative-control probes *a4-a6* indicate that the weak-to-moderate signals produced by tiling probes *t22, t24,* and *t25* that overlap the exon 2 sequence are probably due to unintended, partially complementary or non-specific hybridization or, in other words, the weak-to-moderate signals of the tiling probes and corresponding negative deletion-negative-control probes reveals a situation such as that shown in Figure 16C. Similarly, the weak signals produced by the array cells containing the linker-jump-negative-control probes *a7-a9* and the deletion-negative-control probes *a10-a12* indicate non-specific or unintended complementary hybridization of the exonic tiling probes and two of the three jump probes to various target subsequences.

Thus, by using negative control probes along with the different types of positive probes within the hypothetical array, the ambiguities with respect to exon 2 are resolved. Of course, in actual microarray-based variant splicing analyses, a great many more negative control features targeted to a great many more potential data ambiguities would normally be employed. The analysis of the negative control probe data may be automated in order to disentangle the many overlapping positive and negative probes related to particular exons. Negative-control-probe data may be subtracted from corresponding positive probe signals in order to produce corrected positive probe signals during analysis.

Although the present invention has been described in terms of a particular embodiment, it is not intended that the invention be limited to this embodiment. Modifications within the spirit of the invention will be apparent to those skilled in the art. For example, in the case that both in-order and reverse-order exon splicing is anticipated, the positive jump probes include reverse-jump-negative-control probes. As another example, the number of deletions employed to construct a negative deletion-negative-control probe from a positive probe may vary depending on the length, and nucleotide subunits, of the positive probe. Positive tiling probes, positive exonic tiling probes, and positive jump probes may all be constructed to have similar nucleotide-subunit lengths, or, alternatively, may be constructed to have particular *t*_{*m*} melting points when hybridized by complementary base pairing to target sequences. Therefore, the negative control probes may also either be of uniform length, or may vary, depending on the length of the corresponding positive probes. The described method for analyzing the presence of variant splicing products may be incorporated within an experimental-data-analysis program that automates determination of variant splicing products from experimental results provided by employing the above-described negative control probes.

The foregoing description, for purposes of explanation, used specific nomenclature to provide a thorough understanding of the invention. However, it will be apparent to one skilled in the art that the specific details are not required in order to practice the invention. The foregoing descriptions of specific embodiments of the present invention are presented for purpose of illustration and description. They are not intended to be exhaustive or to limit the invention to the precise forms disclosed. Obviously many modifications and variations are possible in view of the above teachings. The embodiments are shown and described in order to best explain the principles of the invention and its practical applications, to thereby enable others skilled in the art to best utilize the invention and various embodiments with various modifications as are suited to the particular use contemplated. It is intended that the scope of the invention be defined by the following claims and their equivalents:

## Claims

1. A method for determining the sequence of a variant splicing product (920) of an initial mRNA transcript (902) composed of exons (922-928) and introns (914-919), the method comprising:
employing different types of positive probes (1102, 1202, 1308) that hybridize with, and produce signals corresponding to, subsequences of the initial mRNA transcript in a sample solution;
employing at least one type of negative control probe (1708, 1804) corresponding to each type of positive probe to produce negative-control-probe signals;
detecting signals (1402-1412) produced from positive probes in order to determine subsequences of the initial mRNA transcript present in a sample solution and construct an initial sequence of the variant splicing product; and
detecting signals (1612, 1912, 1914, 1916, 1918, 1920, 1922) produced from the negative control probes to resolve ambiguities in the initial sequence of the variant splicing product.

2. The method of claim 1 wherein the different types of positive probes include:
positive tiling probes (1102) complementary to subsequences that span the sequence of the initial mRNA transcript;
positive exonic tiling probes (1202) complementary to exon sequences within the initial mRNA transcript; and
positive jump probes (1308), each jump probe complementary to a subsequence including a potential splice point between two exons of the initial mRNA transcript.

3. The method of claim 1 wherein negative control probes include:
deletion-negative-control probes *(t*_{*x*}*'* in Figure 16A) produced by deleting nucleotide monomers at intervals from positive tiling probes and positive exonic tiling probes;
reverse-jump-negative-control probes (1708), each reverse-jump-negative-control probe having, as a first subsequence, a second subsequence of a corresponding positive jump probe and having, as a second subsequence, a first subsequence of the corresponding positive jump probe, the first subsequence of the corresponding positive jump probe preceding the splice point, and the second subsequence of the corresponding positive jump probe following the splice point;
exon-linker-negative-control probes (1804), including a linking, repeat sequence and a subsequence of a corresponding positive jump probe; and
intron/exon-negative-control probes (1904), including a subsequence that spans the junction between an exon and an intron in the initial mRNA transcript.

4. The method of claim 3 further including:
comparing a signal (1612) detected from a deletion-negative-control probe to the signal detected from a corresponding positive probe (1402-1412);
when the signal detected from the deletion-negative-control probe is comparable in signal strength to the signal detected from a corresponding positive probe, determining that the signal detected from the corresponding positive probe was generated by one of:
non-specific association of the corresponding positive probe with non-fully-complementary target molecules;
non-specific association of the corresponding positive probe with non-complementary target molecules;
experimental error;
instrumental error; and
contamination.

5. The method of claim 3 further including:
comparing a signal detected from a reverse-jump-negative-control probe (1708) to the signal detected from a corresponding positive jump probe (1402-1412);
when the signal detected from the reverse-jump-negative-control probe is comparable in signal strength to the signal detected from a corresponding positive jump probe, determining that the signal detected from the corresponding positive jump probe was generated by one of:
non-specific association of the corresponding positive jump probe with non-fully-complementary target molecules;
non-specific association of the corresponding positive jump probe with non-complementary target molecules;
experimental error;
instrumental error; and
contamination.

6. The method of claim 3 further including:
comparing a signal detected from an intron/exon-negative-control probe (1904) to the signal detected from a corresponding positive jump probe (1402-1412);
when the signal detected from the intron/exon-negative-control probe is greater in signal strength to the signal detected from a corresponding positive jump probe, determining that an exon/exon splice point to which the positive jump probe is complementary is probably not present in the variant splicing product;
when the signal detected from the intron/exon-negative-control probe is smaller in signal strength than the signal detected from a corresponding positive jump probe, determining that an exon/exon splice point to which the positive jump probe is complementary is probably present in the variant splicing product; and
when the signal detected from the intron/exon-negative-control probe is comparable in signal strength to the signal detected from a corresponding positive jump probe, determining that the signal detected from the corresponding positive jump probe was generated by one of:
non-specific association of the corresponding positive jump probe with non-fully-complementary target molecules;
non-specific association of the corresponding positive jump probe with non-complementary target molecules;
experimental error;
instrumental error; and
contamination.

7. Computer instructions that implement a method as claimed in any preceding claim encoded in a computer readable data-storage medium.

8. A microarray (520) manufactured for use in identifying variant splicing products (920) of an initial mRNA transcript, the microarray comprising:
a substrate; and
an active surface of the substrate onto which features containing probe molecules (504-509) are deposited, the probe molecules including:
positive probes (1102, 1202, 1308) complementary to expected subsequences of variant splicing products of the initial mRNA transcript; and
two or more different types of negative control probes (1708, 1804, 1904).

9. The microarray of claim 9 wherein positive probes include:
positive tiling probes (1102) complementary to subsequences that span the sequence of the initial mRNA transcript;
positive exonic tiling probes (1202) complementary to exon sequences within the initial mRNA transcript; and
positive jump probes (1308), each jump probe complementary to a subsequence including a potential splice point between two exons of the initial mRNA transcript.

10. The microarray of claim 9 wherein negative control probes include:
deletion-negative-control probes *(t*_{*x*}*'* in Figure 16A) produced by deleting nucleotide monomers at intervals from positive tiling probes and positive exonic tiling probes;
reverse-jump-negative-control probes (1708), each reverse-jump-negative-control probe having, as a first subsequence, a second subsequence of a corresponding positive jump probe and having, as a second subsequence, a first subsequence of the corresponding positive jump probe, the first subsequence of the corresponding positive jump probe preceding the splice point, and the second subsequence of the corresponding positive jump probe following the splice point;
exon-linker-negative-control probes (1804), including a linking, repeat sequence and a subsequence of a corresponding positive jump probe; and
intron/exon-negative-control probes (1904), including a subsequence that spans the junction between an exon and an intron in the initial mRNA transcript.
